# EUROPÄISCHE PATENTSCHRIFT

(11) **EP 2 295 398 B1**
(45) Veröffentlichungstag und Bekanntmachung des Hinweises auf die Patenterteilung: **13.04.2016**
(21) Anmeldenummer: 10011988.2
(22) Anmeldetag: 19.08.2004
(51) Int. Cl.: C07C 209/68, C07F 9/54, C07C 381/12, C07C 211/62

(54) **Verfahren zur Herstellung ionischer Flüssigkeiten, ionischer Feststoffe oder Gemische derselben**
Method for producing ionic liquids, ionic solids or mixtures thereof
Procédé pour produire des liquides ioniques, des solides ioniques ou leurs melanges

(30) Priorität: 27.08.2003 AT 13512003
(43) Veröffentlichungstag der Anmeldung: 16.03.2011
(62) Teilanmeldung aus: 04764280.6
(73) Patentinhaber: proionic GmbH & Co KG, 8074 Grambach (AT)
(72) Erfinder: Kotschan, Michael, 2100 Korneuburg (AT); Kalb, Roland, 8700 Leoben (AT)
(74) Vertreter: Vinazzer, Edith

(56) Entgegenhaltungen:
- EP-A- 1 182 196
- WO-A-02/079212
- WO-A-03/022812
- M. VISCONTINI ET AL.: "Synthese einiger Methylimino-diessigsäurederivate", HELV. CHIM. ACTA 35, 1952, Seiten 451-455, XP009044807,
- E. ALCALDE ET AL.: "Novel Bis-betaines and Betaines within [1four]meta-Heterophane Frameworks", CHEM. EUR. J., Bd. 8, Nr. 2, 2002, Seiten 474-484, XP009044815,
- J. HOWITZ ET AL.: "Ueber p-Oxy-chinolone und einige Halogenalkylate des ana-Brom-p-Oxychinolins", CHEM. BER. 38, 1905, Seite 888, XP009044883,

## Beschreibung

Die Erfindung betrifft ein Verfahren zur Herstellung ionischer Flüssigkeiten, ionischer Feststoffe oder Gemische derselben aus kationischen Synthesemodulen [Q⁺]ₙ[Yⁿ⁻] und anionischen Synthesemodulen.

Ionische Verbindungen - Verbindungen, welche ionische Flüssigkeiten oder ionische Feststoffe sind - weisen äußerst interessante Eigenschaften auf, wie beispielsweise einen nicht messbaren Dampfdruck, einen sehr großen Liquidusbereich, gute elektrische Leitfähigkeit und ungewöhnliche Solvatations-Eigenschaften. Diese Eigenschaften prädestinieren sie für den Einsatz in verschiedenen Bereichen technischer Anwendungen. So können sie beispielsweise als Lösungsmittel (bei organischer und anorganischer Synthese im Allgemeinen, bei der Übergangsmetallkatalyse, der Biokatalyse, der Phasentransfer-Katalyse, bei Mehrphasen-Reaktionen, in der Photochemie, in der Polymersynthese und der Nanotechnologie), als Extraktionsmittel (bei der flüssig-flüssig- und der flüssig-gasförmigen-Extraktion im Allgemeinen, der Entschwefelung von Rohöl, der Entfernung von Schwermetallen aus Abwässern, der Flüssigmembranextraktion), als Elektrolyte (in Batterien, Brennstoffzellen, Kondensatoren, Solarzellen, Sensoren, in der Elektrochromie, der Galvanotechnik, in der elektrochemischen Metallbearbeitung, in der elektrochemischen Synthese im Allgemeinen, bei der elektroorganischen Synthese, der Nanotechnologie), als Schmierstoffe, als Thermofluide, als Gele, als Reagenzien zur organischen Synthese, in der "Green Chemistry" (Ersatz für Volatile Organic Compounds), als Antistatika, in Spezialanwendungen der Analytik (Gaschromatographie, Massenspektroskopie, Kapillarzonenelektrophorese), als Flüssigkristalle, etc. eingesetzt werden. Diesbezüglich wird beispielsweise auf "Rogers, Robin D.; Seddon, Kenneth R. (Eds.); lonic Liquids - Industrial Applications to Green Chemistry, ACS Symposium Series 818, 2002; ISBN 0841237891" und "Wasserscheid, Peter; Welton, Tom (Eds.); lonic Liquids in Synthesis, Verlag Wiley-VCH 2003; ISBN 3527305157" verwiesen.

In der WO 02/079212 A2 sind Phosphonium-Phosphinat-Verbindungen und ihre Herstellungsverfahren offenbart. Phosphoniumsalze sind ionische Flüssigkeiten, die als polare Lösungsmittel verwendet werden. Die EP 1 182 196 A1 betrifft ein Verfahren zur Herstellung ionischer Flüssigkeiten der allgemeinen Formel [A]n⁺[Y]ⁿ⁻, n=1 oder 2, durch Alkylierung der zugrundeliegenden Amine, Phosphine, Imidazole, Pyrazole, Triazole oder Pyridine mit einem Disulfat der allgemeinen Formel R⁴-SO₄-R⁵ und nachfolgendem und Austausch des Sulfatanions.

Die Optimierung der Eigenschaften für die jeweilige Anwendung kann in weiten Grenzen durch eine Variation der Struktur von Anion und Kation bzw. eine Variation ihrer Kombination erfolgen, was den ionischen Flüssigkeiten die Bezeichnung "Designer Solvents" (siehe beispielsweise Freemantle, M.; Chem. Eng. News, 78, 2000, 37) eingebracht hat. In diesem Sinne wurden viele unsystematische Einzelsynthesen für die Herstellung ionischer Verbindungen im Labor- und Technikumsmaßstab entwickelt. Dementsprechend sind die Kosten der Produktion dieser Verbindungen extrem hoch und die Umsetzbarkeit im Industriemaßstab kaum realisierbar.

Die Produktion ionischer Flüssigkeiten ist bis dato beinahe ausschließlich im Labor- bzw. Technikumsmaßstab möglich, wobei es keine flexible, allgemein anwendbare und effiziente Synthesemethode gibt und insbesondere für beinahe jedes gewünschte Anion ein spezielles Verfahren zur Anwendung kommt. Ausgangsverbindungen dieser Synthesen sind im Allgemeinen alkyl- oder arylsubstituierte organische Stickstoff-, Phosphor- oder Schwefelnucleophile (Amine, Phosphine, Sulfide, Heteroaromaten). Die derzeit bekannten Synthesemethoden werden am Beispiel der Synthese quaternärer Ammoniumverbindungen (siehe nachstehende Abbildung 1) erläutert:

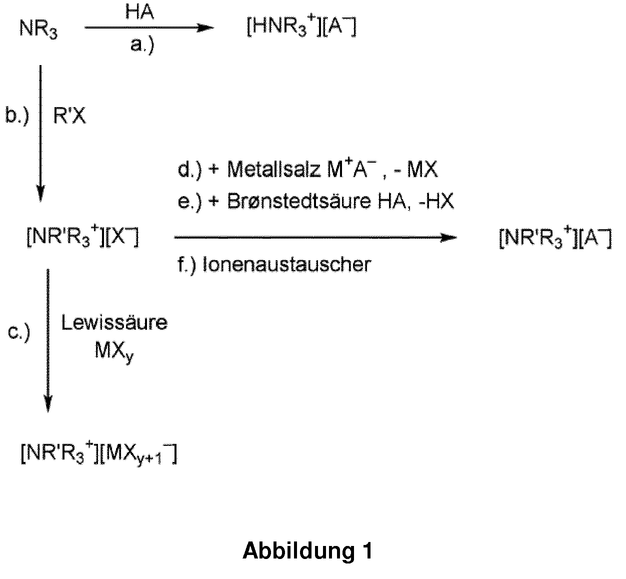

### a. Brønstedt-Säure/Basenreaktion:

Ein Amin R₃N reagiert mit einer Protonensäure HA unter Bildung des Salzes [R₃HN⁺][A⁻]. Diese Darstellung ionischer Flüssigkeiten ist die älteste bekannte (Walden, P., Bull. Acad. Imper. Sci. (St. Petersburg), 1914, 1800) und führt beispielsweise bei der Reaktion von Ethylamin mit Salpetersäure zur Bildung von Ethylammoniumnitrat mit einem Schmelzpunkt von Fₚ = 13°C. In diesem Fall ist das Kation nicht quaterniert sondern nur protoniert. Diese organischen Salze kommen für typische Anwendungsgebiete ionischer Flüssigkeiten nicht in Frage, weil sie thermisch und chemisch instabil sind. Sie werden hier nur der Vollständigkeit halber erwähnt und sind nicht Gegenstand der vorliegenden Erfindung.

### b. Alkylierung bzw. Arylierung zu einem quaternären Salz:

Das Amin R₃N wird mit einem Alkylierungs- oder Arylierungsreagens R'X in einer nucleophilen Substitution unter Bildung eines quaternierten Ammoniumsalzes [NR'R₃⁺][X⁻] umgesetzt (Wilkes, J.S.; Zaworotko, M.J., J. Chem. Soc., Chem. Commun., 13, 1992, 965). Als typische Alkylierungsmittel kommen dabei Haloalkane wie z.B. 1-Chlorbutan, 1-Bromethan, Methyliodid oder Dialkylsulfate wie beispielsweise Dimethylsulfat oder Diethylsulfat zur Anwendung. Diese Alkylierungsmittel sind zwar reaktiv und bilden rasch das gewünschte Produkt, sie sind aber wie alle starken Alkylierungsreagenzien relativ toxisch, zum Teil cancerogen und im Falle der Haloalkane auch atmosphärenschädigend (stratosphärisches Ozon). Die gebildete ionische Verbindung [NR'R₃⁺][X⁻] kann bereits eine Ionische Flüssigkeit sein (Fp < 100°C), dient aber in der Regel als Ausgangsmaterial der folgenden Reaktionen c - f. Sie ist im allgemeinen stark hygroskopisch.

### c. Reaktion mit einer Lewis-Säure:

Zur Darstellung von ionischen Flüssigkeiten auf der Basis von Halometallat-Anionen (siehe z.B. Wilkes, J.S.; Levisky, J.A.; Wilson, R.A.; Hussey, C.L., Inorg. Chem. 1982, 21, 1263) kann das quaternäre Ammoniumsalz [NR'R₃⁺[X⁻] direkt mit einer entsprechenden Lewis-Säure (Elektronenpaarakzeptor) MX_{y} zur gewünschten Verbindung [NR'R₃⁺][MX_{y+1}⁻] umgesetzt werden. Im Falle des Tetrachloroaluminat-Anions [AlCl₄]⁻ wird z.B. ein entsprechendes Ammoniumchlorid [NR'R₃⁺[Cl⁻] direkt mit Aluminiumchlorid AlCl₃ versetzt.

Auf Halometallat basierende ionische Flüssigkeiten sind extrem wasser- und sauerstoff-empfindlich, sehr korrosiv und werden nur für ganz spezielle Zwecke eingesetzt (beispielsweise Olefin-Oligomerisierung, Friedel-Crafts Reaktionen). Bereits geringste Spuren Wasser führen zur Hydrolyse unter Bildung von HCl, HBr oder Hl. Ihre Synthese gestaltet sich dementsprechend schwierig, nicht zuletzt auch aufgrund der Tatsache, dass das Ausgangsmaterial [NR'R₃⁺][X⁻] stark hygroskopisch ist. Sie werden hier nur der Vollständigkeit halber erwähnt und sind nicht Gegenstand der vorliegenden Erfindung.

### d. Anionenaustausch via Metathese:

Das durch Alkylierung bzw. Arylierung gebildete quaternäre Ammoniumsalz [NR'R₃⁺][X⁻] (siehe b.) trägt im Allgemeinen nicht das gewünschte Anion. Um das Halogenid- oder Sulfat-Anion [X⁻] gegen das gewünschte Anion [A⁻] auszutauschen, wird das Salz [NR'R3⁺][X⁻] in einem trockenen, organischen Lösungsmittel (z.B. Aceton, Acetonitril, Methylenchlorid) mit einer stöchiometrischen Menge eines Metallsalzes (meist ein entsprechendes Alkali- oder Erdalkalisalz) [M⁺][A⁻] versetzt, welches das gewünschte Anion [A⁻] aufweist. Der Reaktionsansatz wird unter Ausschluss von Luftfeuchtigkeit typischerweise für einige Tage bis Wochen kräftig gerührt, wobei die gewünschte ionische Flüssigkeit in dem gewählten Lösungsmittel löslich ist und das Anion [X⁻] als unlösliches, festes Metallsalz [M⁺][X⁻] ausfällt und durch Filtration entfernt wird. Neben der sehr langen Reaktionszeit und den großen Mengen von Abfall [M⁺][X⁻] ist bei diesem Verfahren von Nachteil, dass man trockene Lösungsmittel verwenden muss, um einen kompletten Umsatz zu erzielen; ansonsten löst sich das zu entfernende Salz [M⁺][X⁻] in größeren Mengen im Lösungsmittel, wobei diese Halogenidspuren den späteren Einsatz der ionischen Flüssigkeit limitieren (Halogenide sind beispielsweise Katalysatorgifte; Entsorgung durch Verbrennen ist problematisch).

Das wasserfreie Arbeiten ist insbesondere im Industriemaßstab problematisch, da die Vorstufe [NR'R₃⁺][X⁻] im Allgemeinen sehr hygroskopisch ist und der Produktionsprozess daher unter Ausschluss jeglicher Luftfeuchtigkeit erfolgen muss.

Die genannten Nachteile könnten zwar durch den Einsatz von wässrigen Lösungsmittelsystemen und Silbersalzen [Ag⁺][A⁻] behoben werden (wobei die auch in Wasser sehr schwerlöslichen Silberhalogenide ausfallen), dies ist allerdings im Industriemaßstab aus ökonomischen Gründen völlig ausgeschlossen.

Ein kürzlich zum Patent angemeldetes einstufiges Produktionsverfahren (Wasserscheid, Peter.; Hilgers, Claus; Boesmann, Andreas; EP1182197 A1 (Solvent Innovation GmbH, Germany), 2002), welches die Reaktionen b und d vereint, kann zwar das Problem des Ausschlusses von Luftfeuchtigkeit zum Teil entschärfen (keine Isolation der hygroskopischen Zwischenstufe [NR'R₃⁺][X⁻] notwendig), allerdings bleiben die Reaktionszeiten nach wie vor äußerst lang, es fällt weiterhin Abfall an und die potentielle Gefahr der Verunreinigung des Endproduktes mit Halogenid-Ionen ist allgegenwärtig; darüber hinaus lassen sich Kondensationsprodukte und Verunreinigungen, die bei der Darstellung der hier nicht isolierten Zwischenstufe entstehen, nur nachträglich unter erheblich größerem Aufwand aus dem fertigen Produkt entfernen.

Allen Anionenaustauschverfahren via Metathese ist aber insbesondere gemeinsam, dass sie nicht allgemein anwendbar sind und nur für ganz bestimmte Kombinationen aus Kationen und Anionen zufriedenstellend funktionieren. Nur wenn die gewünschte ionische Flüssigkeit im Lösungsmittel ausreichend löslich ist (kann oft problematisch sein, zum Teil große Mengen Lösungsmittel nötig) und/oder das eingesetzte Metallsalz [M⁺][A⁻] im Lösungsmittel wesentlich besser löslich ist als das zu entfernende, kann mit Erfolg gerechnet werden. Des weiteren kann das ausgefällte Salz [M⁺][X⁻] zum Teil in der ionischen Flüssigkeit selbst löslich sein.

### e. Anionenaustausch via Bronstedt-Säure:

Eine weitere Variante des Anionenaustausches stellt die Reaktion der ionischen Vorstufe [NR'R₃⁺][X⁻] mit einer Brønstedtsäure HA dar. In diesem Fall muss die zugehörige freie Säure des gewünschten Anions stärker sein als die entsprechende Säure HX, sodass auch diese Reaktion nur für wenige Anionen [A⁻] in Frage kommt. Ist HX - wie im Falle der Halogenwasserstoffsäuren - eine flüchtige Säure, kann diese verhältnismäßig leicht im Vakuum entfernt werden (ionische Flüssigkeiten besitzen im Allgemeinen keinen messbaren Dampfdruck); ist die ionische Flüssigkeit
a. hydrophob, kann die Säure HX mit Wasser extraktiv entfernt werden. In allen anderen Fällen gestaltet sich die Isolation des Produktes schwierig.
b. **Anionenaustausch an einem lonentauscherharz:**
   Der Austausch des Ions [X] gegen das gewünschte Ion [A⁻] kann auch in einem allgemein bekannten Verfahren an einem herkömmlichen Anionentauscherharz erfolgen; aus ökonomischer Sicht ist dieses Verfahren im Industriemaßstab aber kaum anwendbar, da die maximale Beladung eines Harzes sehr beschränkt ist.

Der Erfindung liegt die Aufgabe zu Grunde, ein Verfahren zur Herstellung ionischer Flüssigkeiten oder ionischer Feststoffe zur Verfügung zu stellen, welches es gestattet, eine sehr breite Palette dieser Substanzen herstellen zu können. Die ionischen Flüssigkeiten oder ionischen Feststoffe sollen in industrierelevanten Mengen "ontime" und kundenspezifisch herstell- und lieferbar sein und beim Hersteller nur einen geringen Lageraufwand erfordern.

Gelöst wird die gestellte Aufgabe erfindungsgemäß dadurch, dass [Q⁺] ein quaterniertes Ammonium- [R¹R²R³R⁴N⁺] Kation ist,
wobei die Reste R¹,R²,R³ und R⁴ lineare, cyclische, verzweigte, gesättigte oder ungesättigte Alkylreste sind und
wobei R¹, R², R³, R⁴ auch untereinander verbunden sein können (mehrbindiger Rest),
wobei [Yⁿ⁻] ein Alkoholat-Anion RO⁻ ist,
wobei R ein linearer, cyclischer, verzweigter, gesättigter oder ungesättigter Alkylrest, ein mono- oder polycyclischer Aromat oder Heteroaromat ist,
wobei das kationische Synthesemodule [Q⁺]ₙ[Yⁿ⁻] in einer Brønstedt-Säure/Base-Reaktion mit einer n-basigen Bronstedt-Säure HₙZ als anionisches Synthesemodul versetzt wird,
wobei das kationische Synthesemodul [Q⁺]n[Yⁿ⁻] in einer Brønstedt-Säure/Base-Reaktion mit einer n-basigen Brønstedt-Säure HₙZ als anionisches Synthesemodul versetzt wird, wodurch das Alkoholat [RO⁻] gegen das gewünschte Anion [Zⁿ⁻) ersetzt wird und als Nebenprodukt der entsprechende Alkohol ROH entsteht.

Die Erfindung besteht in einem innovativen modularen Produktionssystem, mit dessen Hilfe sehr schnell und einfach aus ionischen Synthesevorstufen, den kationischen Synthesemodulen, in Kombination mit geeigneten marktüblichen Handelschemikalien, den anionische Synthesemodulen, eine extrem breite Palette an ionischen Verbindungen hergestellt werden kann, so dass eine ontime-Lieferung von industrierelevanten Mengen ermöglicht wird. Die Erfindung gestattet daher eine modulare Produktion ionischer Verbindungen bzw. deren Gemische über Vorstufen (kationische Synthese module) und deren Reaktion mit Handelschemikalien oder anderen lagerfähigen Zwischenprodukten (anionische Synthesemodule) in einem schnellen Syntheseschritt, der unmittelbar vor der Auslieferung erfolgen kann. Die Erfindung ermöglicht die systematische Bereitstellung einer größeren Produktpalette durch wenige Vorstufen bevorzugt in Verbindung mit ontimelieferbaren Chemikalien- Das Herstellverfahren ist zudem ausgesprochen umweltfreundlich und in der Lage ionische Verbindungen nach Kundenwunsch und "maßgeschneidert" zur Verfügung zu stellen.

Dabei können die kationischen Synthesemodule lagerfähige Vorstufen sein, welche insbesondere zu einem späteren Zeitpunkt mit anionischen Synthesemodulen in einem modularen Produktionsprozess zu ionischen Verbindungen umgesetzt werden. Der Lageraufwand beschränkt sich daher auf universell einsetzbare Vorstufen, was angesichts kaum überschaubarer Kombinationsmöglichkeiten ein äußerst ökonomisches und flexibles System darstellt.

Dieses Verfahren ist vor allem dann von Vorteil, wenn diese Alkoholate [Q⁺][RO⁻] bereits stabile Verbindungen sind, die direkt als kationische *Synthesemodulen* umgesetzt werden, wobei das Alkoholat gegen das gewünschte Anion (Z⁻] ersetzt wird und dabei zum entsprechenden Alkohol ROH reagiert, wobei dann der so entstehende Alkohol destillativ vom Produkt [Q⁺][Z⁻] abgetrennt wird.

Das kationische Synthesemodul [Q+]ₙ[Yⁿ⁻] wird gemäß der Erfindung in einer Brønstedt-Säure/Base-Reaktion mit einer n-basigen Brønstedt-Säure HₙZ als anionisches Synthesemodul versetzt, *wodurch das Anion [Yⁿ⁻] gegen das gewünschte Anion [Zⁿ⁻] ersetzt wird und als Nebenprodukt Wasser der entsprechenden Alkohole ROH entsteht.*

Die Zugabe der n-basigen Brønstedt-Säure HₙZ erfolgt in Substanz oder gelöst in Wasser und/oder anderen Lösungsmitteln stöchiometrisch oder im Überschuss, wobei insbesondere flüchtige Säuren HₙZ bevorzugt im Überschuss, zugegeben werden. Die Zugabe der n-basigen Brønstedt-Säure HₙZ kann auch in einer solchen Stöchiometrie erfolgen, dass nicht das vollständig dissoziierte konjugierte Anion [Zⁿ⁻] entsteht, sondern die zugehörigen protonierten Anionen [HₘZ^{(n-m)-}], m < n.

Die Reaktion wird in wässrigem, wässrig/organischem, organischem Lösungsmittel (Zugabe von polaren Lösungsmitteln wie niederen Alkoholen, Ketonen, Sulfoxiden, Ethern, Nitrilen etc.) ader - im Falle dass das kationische Synthesemodul [Q⁺]ₙ[Yⁿ⁻] bei der gewählten Temperatur flüssig ist - auch lösungsmittelfrei durchgeführt.

Weitere Merkmale, Vorteile und Einzelheiten der Erfindung ergeben sich aus der nachfolgenden Beschreibung. Einige der verwendeten Begriffe sind dabei wie folgt zu verstehen:
**Ionische Flüssigkeiten**: Ionische Flüssigkeiten sind - im Sinne der anerkannten Literatur (Wasserscheid, Peter; Welton, Tom (Eds.); Ionic Liquids in Synthesis, Verlag Wiley-VCH 2003; ISBN 3-527-30515-7)- flüssige organische Salze oder Salzgemische bestehend aus organischen Kationen und organischen oder anorganischen Anionen, mit Schmelzpunkten von unter 100°C.
**Ionische Feststoffe:** Der Begriff ionisch Feststoffe" beinhaltet in dieser Anmeldung Salze im Sinne der ionischen Flüssigkeiten, mit Schmelzpunkten von 100°C und höher. Es besteht neben diesem definitionsgemäß festgelegten Schmelzpunktbereich und dem damit verbundenen Aggregatzustand kein prinzipieller chemischer oder physikalischer Unterschied zu ionischen Flüssigkeiten.
**Ionische Verbindungen:** Sind alle ionischen Flüssigkeiten und ionischen Feststoffe im Sinne der oben angeführten Definitionen.
**Kationische Synthesemodule:** Sind die zur Produktion ionischer Verbindungen eingesetzten, bevorzugt lagerfähigen ionischen Vorstufen [Q⁺]ₙ[Yⁿ⁻], die das gewünschte Kation [Q⁺] aufweisen.
**Anionische Synthesemodule:** Sind die mit kationischen Synthesemodulen zur Reaktion gebrachten, marktüblichen Handelschemikalien oder anderen bevorzugt lagerfähigen Zwischenprodukte, die das Anion [Yⁿ⁻] der kationischen Synthesemodule entfernen und gegen das gewünschte Anion [Zⁿ⁻] der Ionischen Verbindung [Q⁺]ₙ[Zⁿ⁻] ersetzen.
**Synthesemodule:** Sind kationische Synthesemodule oder anionische Synthesemodule.
**Modularer Produktionsprozess:** Definiert die maßgeschneiderte Produktion ionischer Verbindungen und deren Gemische durch Kombination entsprechender kationischer und anionischer Synthesemodule (Baukastenprinzip).

Die Erfindung betrifft ein äußerst vielseitiges und beinahe universell anwendbares Produktionsverfahren zur Herstellung von ionischen Flüssigkeiten und ionischen Feststoffen. Die betreffenden ionischen Verbindungen der allgemeinen Formel [Q⁺]ₙ[Zⁿ⁻] werden in diesem neuen Verfahren in einem - im Gegensatz zu herkömmlichen Verfahren - allgemein anwendbaren, raschen und quantitativen Syntheseschritt aus einer bereits ionischen Vorstufe [Q⁺]ₙ[Yⁿ⁻] erzeugt. Das betreffende Kation [Q⁺]ₙ ist ein quaterniertes Ammonium-[R¹R²R³R⁴N⁺]-Kation, wobei die Reste R¹, R², R³, R⁴ alle gleich, teilweise gleich oder unterschiedlich sein können. Diese Reste R¹, R², R³ und R⁴ können lineare, cyclische, verzweigte, gesättigte oder ungesättigte Alkylreste, mono- oder polycyclische aromatische oder heteroaromatische Reste sein, wobei R¹, R², R³, R⁴ auch untereinander verbunden sein können (mehrbindiger Rest).

Beim erfindungsgemäßen Verfahren zur Herstellung ionischer Verbindungen werden die entsprechenden Alkoholate [Q⁺][RO⁻] direkt als kationische Synthesemodule in einem modularen Produktionsverfahren in einer allgemein bekannten Brønstedt-Säure/Base-Reaktion mit einer n-basigen Brønstedt-Säure HₙZ als anionisches Synthesemodul umgesetzt, wobei das Alkoholat-Anion gegen das gewünschte Anion [Z⁻] ersetzt wird und zum entsprechenden Alkohol ROH reagiert, wobei dann der so entstehende Alkohol ROH destillativ vom Produkt [Q⁺][Z⁻] abgetrennt wird.

Die Reaktion kann dabei batchweise, semibatchweise oder kontinuierlich ausgeführt werden. Sie findet im Allgemeinen in wässriger oder wässrig-organischer Lösung (unter Zugabe von polaren Hilfslösungsmitteln wie niederen Alkoholen, Ketonen, Sulfoxiden, Ethern, Nitrilen etc.) statt, kann aber, im Falle dass die ionische Vorstufe [Q⁺]ₙ[Yⁿ⁻] eine Flüssigkeit ist, auch lösungsmittelfrei ausgeführt werden. Die Zugabe der n-basigen Brønstedt-Säure HₙZ kann in Substanz oder gelöst in Wasser und/oder in anderen Lösungsmitteln stöchiometrisch oder im Überschuss erfolgen, wobei insbesondere flüchtige Säuren HₙZ bevorzugt im Überschuss zugegeben werden, da sich diese später leicht im Vakuum entfernen lassen (ionische Verbindungen besitzen im Allgemeinen keinen messbaren Dampfdruck). Im Falle einer mehrbasigen Säure kann die Zugabe der n-basigen Brønstedt-Säure HₙZ auch in einer solchen Stöchiometrie erfolgen, dass nicht das vollständig dissoziierte konjugierte Anion [Zⁿ⁻] entsteht, sondern auch die zugehörigen protonierten Anionen [HₘZ^{(n-m)-}] entstehen (im Falle der Phosphorsäure H₃PO₄ wären dies beispielsweise das Dihydrogenphosphat [H₂PO₄⁻] und das Hydrogenphosphat [HPO₄²⁻]). Die Säure/Basenreaktion kann durch Anlegen von Vakuum und/oder Erwärmung beschleunigt werden, wodurch eine Erhöhung der Reaktionsgeschwindigkeit und kontinuierliche Entfernung vom entstehenden Alkohol ROH erfolgt.

Die große Vielzahl der möglichen Brønstedt-Säuren HₙZ, die aus einer einzigen, bestimmten ionischen Vorstufe [Q⁺]ₙ[Yⁿ⁻] einfach, kostengünstig, effizient und in hoher Qualität eine Vielzahl von möglichen ionischen Verbindungen [Q⁺]ₙ[Zⁿ⁻] quasi in einem "Baukastensystem" zugänglich machen, prägten den oben erwähnten Begriff "modularer Produktionsprozess", der sich von allen bis dato publizierten konventionellen Produktionsprozessen deutlich abhebt, siehe beispielsweise Wasserscheid, Peter; Welton, Tom (Eds.); "Ionic Liquids in Synthesis, 2nd Edition", Verlag Wiley-VCH 2008; ISBN 978-3-527-31239-9. Die dadurch zugänglichen Anionen [Zⁿ⁻] und [HₘZ^{(n-m)-}] beschränken sich nicht auf die wenigen bis jetzt üblichen Anionen wie beispielsweise Tetrafluoroborat [BF₄⁻], Hexafluorophosphat [PF₆⁻],Bis-(trifluormethylsulfonyl)imid [(CF₃SO₂)₂N⁻], Trifluormethansulfonat [CF₃SO₂⁻], Trifluoracetat [CF₃CO₃⁻], Methansulfonat [CH₃SO₂⁻], Acetat, Chlorid, Bromid oder Alkylsulfat.

## Patentansprüche

1. Verfahren zur Herstellung ionischer Flüssigkeiten, ionischer Feststoffe oder Gemische derselben aus kationischen Synthesemodulen [Q⁺]ₙ[Yⁿ⁻] und anionischen Synthesemodulen,
wobei [Q⁺] ein quaterniertes Ammonium- [R¹R²R³R⁴N⁺] Kation ist,
wobei die Reste R¹, R², R³ und R⁴ lineare, cyclische, verzweigte, gesättigte oder ungesättigte Alkylreste sind und
wobei R¹, R², R³, R⁴ auch untereinander verbunden sein können (mehrbindiger Rest),
wobei [Yⁿ⁻] ein Alkoholat-Anion RO⁻ ist,
wobei R ein linearer, cyclischer, verzweigter, gesättigter oder ungesättigter Alkylrest, ein mono- oder polycyclischer Aromat oder Heteroaromat ist,
wobei das kationische Synthesemodul [Q⁺]ₙ[Yⁿ⁻] in einer Brønstedt-Säure/Base-Reaktion mit einer n-basigen Bronstedtø-Säure HₙZ als anionisches Synthesemodul versetzt wird, wodurch das Alkoholatanion [RO⁻] gegen das gewünschte Anion [Zⁿ⁻] ersetzt wird und als Nebenprodukt der entsprechende Alkohol ROH entsteht

2. Verfahren nach Anspruch 1, **dadurch gekennzeichnet, dass** die Zugabe der n-basigen Brønstedt-Säure HₙZ in Substanz oder gelöst in Wasser und/oder anderen Lösungsmitteln stöchiometrisch oder im Überschuss erfolgt, wobei insbesondere flüchtige Säuren HₙZ bevorzugt im Überschuss, zugegeben werden.

3. Verfahren nach Anspruch 1 oder 2, **dadurch gekennzeichnet, dass** die Zugabe der n-basigen Brønstedt-Säure HₙZ auch in einer solchen Stöchiometrie erfolgen kann, dass nicht das vollständig dissoziierte konjugierte Anion [Zⁿ⁻] entsteht, sondern die zugehörigen protonierten Anionen [HₘZ^{(n-m)-}], m < n.

4. Verfahren nach einem der Ansprüche 1 bis 3, **dadurch gekennzeichnet, dass** die Reaktion in wässrigem, wässrig/organischem, organischem Lösungsmittel (Zugabe von polaren Lösungsmitteln wie niederen Alkoholen, Ketonen, Sulfoxiden, Ethern, Nitrilen) oder - im Falle dass das kationische Synthesemodul [Q⁺]ₙ[Yⁿ⁻] bei der gewählten Temperatur flüssig ist - auch lösungsmittelfrei durchgeführt wird.

5. Verfahren nach Anspruch 1, **dadurch gekennzeichnet, dass** kationische Synthesemodule mit anionischen Synthesemodulen in einem modularen Produktionsprozess zu ionischen Verbindungen umgesetzt werden.

6. Verfahren nach Anspruch 1 oder 5, **dadurch gekennzeichnet. dass** die kationische Synthesemodule erst unmittelbar vor der Produktion der ionischen Verbindung mit den anionischen Synthesemodulen umgesetzt werden.

## Claims

1. Method for producing ionic fluids, ionic solids or mixtures of the same from cationic synthesis modules [Q⁺]ₙ[Yⁿ⁻] and anionic synthesis modules,
wherein [Q⁺] is a quaternated ammonium [R¹R²R³R⁴N⁺] cation,
wherein the radicals R¹, R², R³ and R⁴ are linear, cyclic, branched, saturated or unsaturated alkyl radicals and
wherein R¹,R², R³, R⁴ can also be interconnected (multiple-bound radical),
wherein [Yⁿ⁻] is an alcoholate anion RO,
wherein R is a linear, cyclic, branched, saturated or unsaturated alkyl radical, a mono- or polycyclic aromatic compound or heteroaromatic compound,
wherein the cationic synthesis module [Q⁺]ₙ[Yⁿ⁻] is mixed with an n-based Brønstedt acid HₙZ in a Brønstedt acid/base reaction as anionics synthesis module, so that the alcoholate anion [RO⁻] is replaced with the desired anion [Zⁿ⁻] and the corresponding alcohol ROH created as a by-product.

2. Method according to claim 1, **characterised in that** the addition of the n-based Brønstedt acid HₙZ takes place in substance or dissolved in water and/or another solvent stochiometrically or in excess, wherein volatile acids HₙZ are preferred in excess.

3. Method according to claim 1 or 2, **characterised in that** the addition of the n-based Brønstedt acid HₙZ can also take place after such stoichiometry, that not the completely disassociated conjugated anion [Zⁿ⁻] is created, but the associated protonated anions [HₘZ^{(n-m)-}], m < n.

4. Method according to one of the claims 1 to 3, **characterised in that** the reaction is carried out in aqueous, aqueous/organic, organic solvent (addition of polar solvents such as low alcohols, ketones, sulfoxides, ethers, nitriles) or - in a case where the cationic synthesis module [Q⁺]_{n[}Yⁿ⁻] is liquid at the selected temperature - also without a solvent.

5. Method according to claim 1, **characterised in that** the cationic synthesis module is converted into ionic compounds with anionic synthesis modules during a modular production process.

6. Method according to claim 1 or 5, **characterised in that** the cationic synthesis module is not converted into the ionic compound with the anionic synthesis modules until directly prior to production.

## Revendications

1. Procédé de préparation de liquides ioniques, de matières solides ioniques ou de leurs mélanges à partir de modules de synthèse cationiques [Q⁺]_{n[}Yⁿ⁻] et de modules de synthèse anioniques,
[Q⁺] étant un cation quaternisé d'ammonium- [R¹R²R³R⁴N⁺],
les radicaux R¹, R², R³ et R⁴ étant des radicaux alkyles saturés ou insaturés de nature linéaire, cyclique, ramifiée et
R¹, R², R³, R⁴ pouvant également être reliés entre eux (radical polyvalent),
[Yⁿ⁻] étant un alcoolate anionique RO⁻,
R étant un radical alkyle saturé ou insaturé de nature linéaire, cyclique, ramifiée, un hétérocycle aromatique de nature mono- ou polycyclique,
sachant que le module de synthèse cationique [Q⁺]ₙ[Yⁿ⁻] est ajouté dans une réaction acide-base selon Brønstedt avec un acide de Brønstedt HₙZ à n bases servant de module de synthèse anionique, l'alcoolate anionique [RO⁻] étant remplacé par l'anion souhaité [Zⁿ⁻] les sous-produits générés étant les alcools ROH correspondants.

2. Procédé selon la revendication 1, **caractérisé en ce que** l'ajout dudit acide Brønstedt HₙZ à n bases est réalisé en masse ou après dissolution dans de l'eau et/ou dans d'autres solvants de façon stoechiométrique ou excédentaire, sachant que des acides HₙZ particulièrement volatiles sont ajoutés de préférence de façon excédentaire.

3. Procédé selon l'une quelconque des revendications 1 ou 2, **caractérisé en ce que** l'ajout dudit acide Brønstedt HₙZ à n bases peut également être réalisé selon une stoechiométrie telle que l'on n'obtient pas l'anion conjugué [Zⁿ⁻] sous sa forme complètement dissociée mais les anions protonés correspondants [HₘZ^{(n-m)-}], m < n.

4. Procédé selon l'une quelconque des revendications 1 à 3, **caractérisé en ce que** ladite réaction est réalisée dans un solvant aqueux, aqueux/organique, organique (ajout de solvants polaires tels que les alcools, cétones, sulfoxydes, éthers, nitriles) ou bien - dans le cas d'un module de synthèse cationique [Q⁺]ₙ[Yⁿ⁻] liquide à la température sélectionnée - également sans ajout de solvant.

5. Procédé selon la revendication 1, **caractérisé en ce que** les modules de synthèse cationiques sont mis en oeuvre avec les modules de synthèses anioniques dans un processus de production modulaire en vue de composés ioniques.

6. Procédé selon l'une quelconque des revendications 1 ou 5, **caractérisé en ce que** les modules de synthèses cationiques sont mis en oeuvre immédiatement avant la production du composé ionique avec les modules de synthèse anioniques.
